# EUROPÄISCHE PATENTSCHRIFT

(11) **EP 1 528 904 B1**
(45) Veröffentlichungstag und Bekanntmachung des Hinweises auf die Patenterteilung: **19.11.2008**
(21) Anmeldenummer: 02752928.8
(22) Anmeldetag: 13.08.2002
(51) Int. Cl.: A61F 5/455

(54) **Einweg-Hilfsmittel zum Urinieren**
Disposable urinary aid
Dispositif perdu d'aide à la miction

(43) Veröffentlichungstag der Anmeldung: 11.05.2005
(73) Patentinhaber: Flückiger, Werner, 5727 Oberkulm (CH)
(72) Erfinder: Flückiger, Werner, 5727 Oberkulm (CH)
(74) Vertreter: Heubeck, Bernhard
(86) Internationale Anmeldenummer: PCT/CH2002/000441
(87) Internationale Veröffentlichungsnummer: WO 2004/014265

(56) Entgegenhaltungen:
- EP-A- 1 078 614
- WO-A-00/15166
- US-A- 2 878 486
- US-A- 5 333 330
- US-B1- 6 202 225

## Beschreibung

Die Erfindung betrifft ein Einweg- Hilfsmittel zum Urinieren gemäss dem Oberbegriff des Anspruches 1.

Die Erfindung geht von einem Hilfsmittel aus, das aus der EP 1 078 614 bekannt ist. Das Hilfsmittel ist als Ableitungsorgan konzipiert und ist aus zwei flach aneinander liegenden Wänden aus einem in Wasser aufweichbaren und/oder zersetzbaren , saugfähigen Hygienepapier gebildet, welche einen Ableitungskanal begrenzen. Das Ableitungsorgan umfasst einen Einlassabschnitt mit einer an den Körper des jeweiligen Benutzers ansetzbaren Randpartie und einen Ableitungsabschnitt mit einer Öffnung zum Ableiten von Urin in eine Toilette. Das Ableitungsorgan ist in der Längserstreckung gefaltet. Die Wände sind an den Seitenrändern als auch am Griffteil miteinander verbunden. Die Wandteile sind miteinander verklebt oder mechanisch miteinander verbunden. Die Wandteile können auch je mit einer quer zur Längserstreckung des Ableitungsorgans verlaufenden Wölbung ausgeführt sein.

Die bekannten Hilfsmittel weisen einen Einlassabschnitt mit aneinander liegenden oder mit gewölbten Wandteilen auf. Zur Herstellung der Verbindung ist die Anwendung von Druck erforderlich, was dazu führt, dass die Wandteile nicht nur aneinander liegen sondern auch aneinander haften. Die Hilfsmittel werden in gerollter oder gestapelter Form einzeln aus einem Spender entnommen. Durch die Wickeldichte und die Packungsdichte wird die Haftung der Wandteile verstärkt und die Wölbung verringert, insbesondere durch eine längere Lagerung.

Als nachteilig wird angesehen, dass der Einlassabschnitt vor Gebrauch durch manuelles Spreizen geöffnet werden muss und bei der Herstellung ein grösserer Abfall anfällt.

Die US-A-2 878 486 offenbart ein Einweg- Hilfsmittel mit einem gefalteten Ableitungsorgan, das aus zwei flach aneinander liegenden Wänden aus einem Hygienepapier gebildet ist. Das Ableitungsorgan besteht aus einem durch je zwei Wandabschnitte jeder der beiden Wände gebildeten, zu einem Trichter verformbaren Kanal zur Aufnahme und zum Ableiten von Urin in eine Toilette und einem aus zwei Wandabschnitten gebildeten, seitlich abstehenden Griffteil, der mit den Wandabschnitten des Kanals einstückig ausgebildet ist. Die Wände sind so gefaltet, dass die Wandabschnitte des Ableitungskanals übereinander liegend angeordnet sind und dass der Griffteil durch die Wandabschnitte vollständig umschlossen ist.

Zum Öffnen des gefalteten Ableitungsorgans, muss zuerst der Griffteil durch manuelles Spreizen der gefalteten Wandabschnitte des Kanals zugänglich gemacht werden, um diese anschliessend entfalten zu können.

Der Erfindung liegt die objektive Aufgabe zugrunde, ein verbessertes und einfacher zu handhabendes Hilfsmittel der eingangs genannten Art zu schaffen, welches die vorstehend genannten Nachteile nicht aufweist.

Diese Aufgabe wird erfindungsgemäss mit den Merkmalen des Anspruchs 1 gelöst.

Das erfindungsgemäss ausgebildete Hilfsmittel enthält ein gegenüber den bekannten Ausführungen verbessertes Ableitungsorgan, das einen doppelt gefalteten Einlassabschnitt aufweist, welcher durch Schwenken des hinausragenden Griffteils automatisch geöffnet werden kann, und das aufgrund des geringen Materialabfalls kostengünstig und automatisch erzeugt werden kann.

Vorteilhafte Ausführungen ergeben sich aus den abhängigen Ansprüchen.

Nachstehend wird die Erfindung anhand der beiliegenden Zeichnungen erläutert. Es zeigen:
- Fig. 1: eine Seitenansicht einer ersten Ausführung eines erfindungsgemässen Hilfsmittel;
- Fig. 2: einen Schnitt entlang der Linie II-II in Fig. 1;
- Fig. 3: eine Aufsicht des Hilfsmittels nach Fig. 1 im Gebrauchszustand;
- Fig. 4: einen Zuschnitt der Ausführung nach Fig. 1;
- Fig. 5 a-c: schematische Darstellungen der Verfahrensschritte zur Herstellung der Ausführung nach Fig. 1;
- Fig. 6: eine modifizierte Form des Hilfsmittels nach Fig. 1;
- Fig. 7: eine zweite Ausführung des erfindungsgemässen Hilfsmittels;
- Fig. 8: einen Schnitt entlang der Linie VIII-VIII in Fig. 7;
- Fig. 9: eine Aufsicht des Hilfsmittels nach Fig. 7 im Gebrauchszustand;
- Fig. 10 a-c: drei Zuschnitte der Ausführung nach Fig. 7;
- Fig. 11: eine schematische Darstellung der Verfahrensschritte zur Zusammenstellung der Ausführung nach Fig. 7;
- Fig. 12: einen Schnitt entlang der Linie XII-XII in Fig. 11 und
- Fig. 13 a-c: eine perspektivische Darstellung der Verfahrensschritte 1 bis 7 zur Zusammenstellung der Ausführung nach Fig. 7.

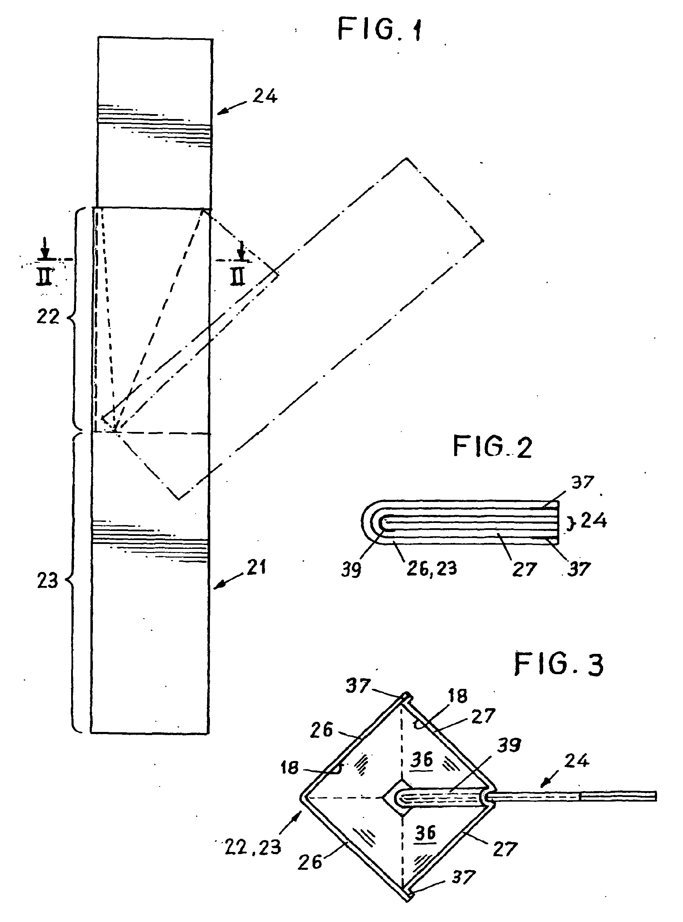

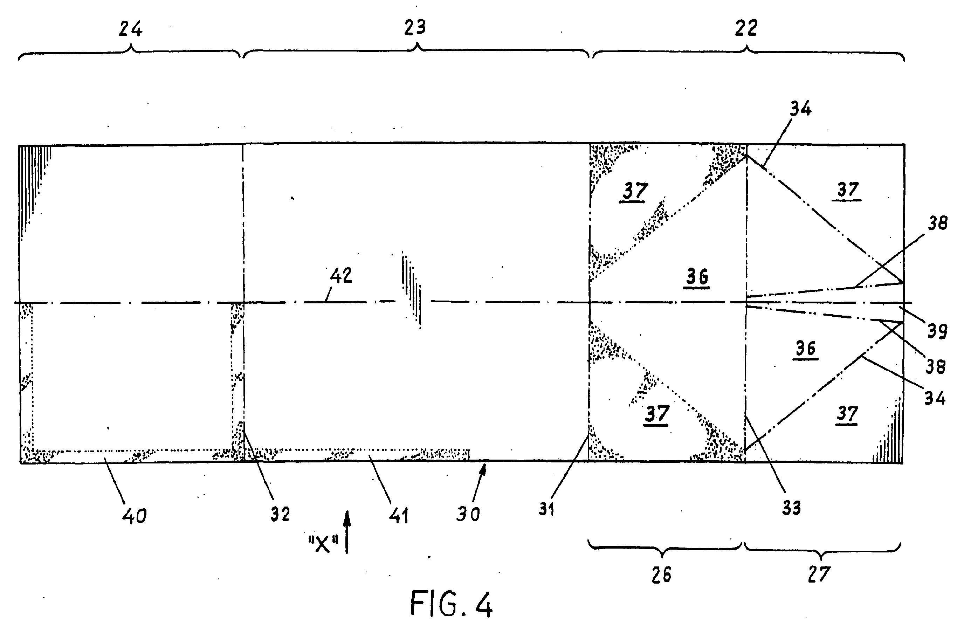

Es wird auf die Fig. 1 bis 6 Bezug genommen, die eine erste Ausführung des Hilfsmittels zeigen. Das Hilfsmittel besteht aus Hygienepapier und ist als Ableitungsorgan 21 ausgebildet, welches einen Einlassabschnitt 22, einen Ableitungsabschnitt 23 und einen Griffteil 24 aufweist. Der Einlassabschnitt 22 ist aus einer ersten wand mit zwei wand abschnitten 26, die einstückig mit zwei wandabschnitten zur Bildung des Ableitungsabschnitts 23 ausgebildet sind und einer zweiten Wand mit zwei Wand-abschnitten 27 gebildet, die je, mit einen der Wand-abschnitte 26 der ersten wand verbunden sind. Der Griffteil 24 ist mit den wandabschnitten 27 der zweiten wand verbunden und ragt über den Einlassabschnitt 22 hinaus. Der Einlassabschnitt 22, der Ableitungsabschnitt 23 und der Griffteil 24 werden aus den in der Fig. 4 dargestellten Zuschnitt gebildet.

Wie die Fig. 4 zeigt, ist der Zuschnitt 30 für das Ableitungsorgan 21 streifenförmig und durch Rillen 31 und 32 in einen Abschnitt zur Bildung des Einlassabschnitts 22, einen Abschnitt zur Bildung des Ableitungsabschnitts 23 und einen Abschnitt zur Bildung des Griffteiles 24 unterteilt. Der Abschnitt zur Bildung des Einlassabschnitts 22 ist durch eine Rille 33 in die Abschnitte 26 und 27 unterteilt. Der Abschnitt 27 ist mit zwei Rillen 34 versehen, welche den Abschnitt 27 jeweils in einen Abschnitt 36 und zwei Abschnitte 37 unterteilen. Der Abschnitt 27 ist ferner mit zwei Rillen 38 versehen, die einen Abschnitt39 zur Befestigung des Griffteils 24 begrenzen.

Die Herstellung des vorstehend beschriebenen Ableitungsorgans erfolgt in Richtung des Pfeiles X mit einem takt-und anschlagfreien Verfahren, welches die folgenden Verfahrensschritte umfasst.

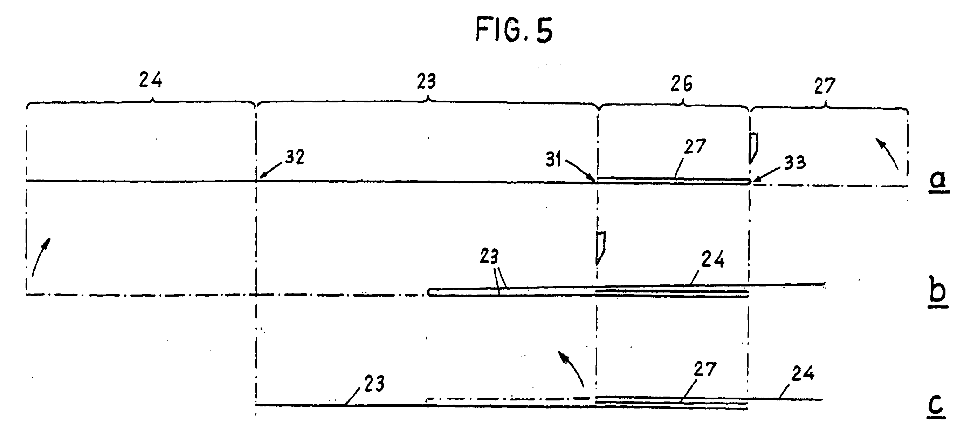

In einem ersten Schritt wird eine Papierbahn 6 kontinuierlich zugeführt, mit den Rillen 31, 32, 33, 34 und 38 versehen und der Zuschnitt 30 ausgestanzt. In einem zweiten Schritt wird auf den Abschnitten 37 des Abschnitts 26 Klebmittel aufgebracht (Fig.4). In einem dritten Schritt wird der Abschnitt 27 entlang der Rille 33 gefaltet, so dass die Abschnitte 26 und 27 aufeinander liegen und danach wird der Faltabschnitt zwischen den Abschnitten 26 und 27 abgetrennt, so dass eine Öffnung entsteht (Fig. 5a). In einem vierten Schritt werden die Abschnitte mittels Druck miteinander verbunden und auf den gefalteten Abschnitt 27 im Abschnitt 39 ein Klebmittel aufgebracht. In einem fünften Schritt wird der Abschnitt 23 so umgelegt, dass die Rille 32 deckungsgleich mit der Rille 31 ist, der Abschnitt 24 abgetrennt und der Abschnitt 23 wieder zurückgelegt (Fig. 5b). In einem sechsten Schritt wird die nun frei liegende Rückseite des Abschnittes 24 im Abschnitt 40 und der Abschnitt 23 im Abschnitt 41 mit einem Klebmittel versehen als auch der Abschnitt 24 gleichzeitig mit dem Abschnitt 27 verbunden (5c). In einem siebenten Schritt wird der gefaltete Zuschnitt entlang der Mittellinie 42 gefaltet, die Abschnitte 23 und 24 aufeinander gelegt und mittels Druck miteinander verbunden. Anstelle der Papierbahn 6 kann auch ein Papierstreifen zugeführt werden.

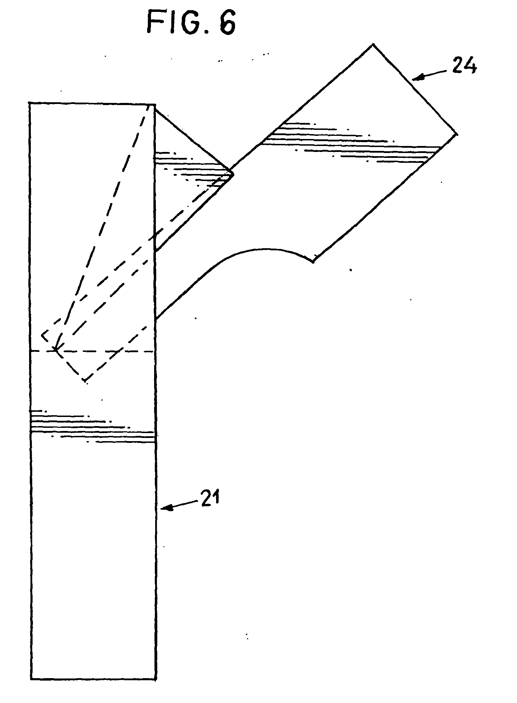

Die Fig.6 zeigt ein Ableitungsorgan 21 mit einem modifizierten Griffteil 24.

Die Hilfsmittel werden ineinander gefächert und in einen Spender verpackt. Der Spender weist eine Öffnung zur Entnahme eines Hilfsmittels auf. Die Öffnung ist so gestaltet, dass der Griffteil 5 bzw. 24 aus dem Spender herausragt und die durch das Falten der Abschnitte 26 und 27 gebildeten Kanten an den die Öffnung des Spenders begrenzenden Wandteilen des Spender anliegen. Dadurch kann mittels des Griffteils 5 bzw. 24 das Hilfsmittel unter gleichzeitiger Öffnung des trichterförmigen Einleitungsabschnitt aus dem Spender entnommen werden. Daraus ergibt sich der Vorteil, dass das Hilfsmittel im gebrauchsfertigen Zustand vorliegt.

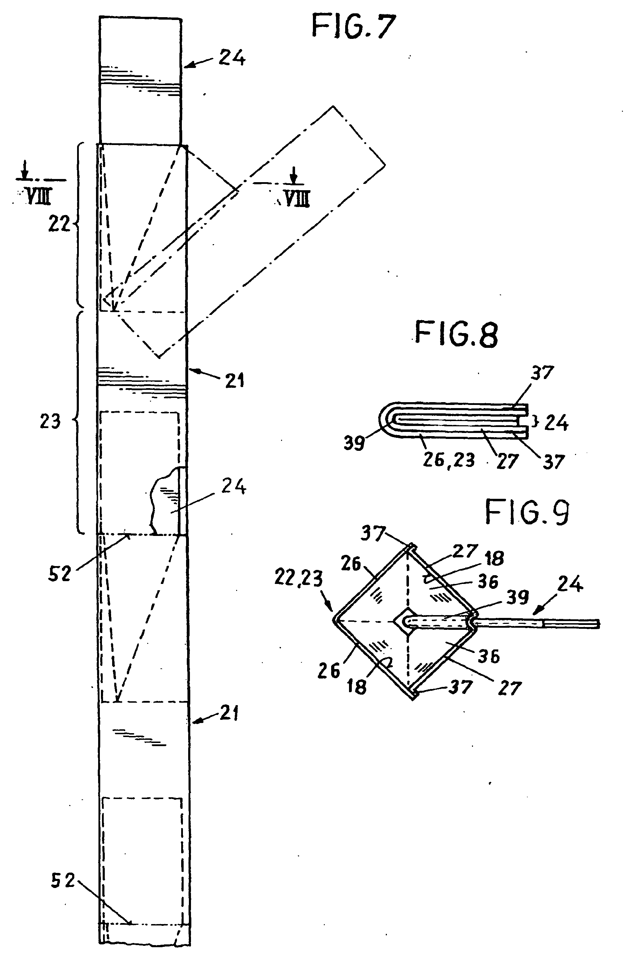

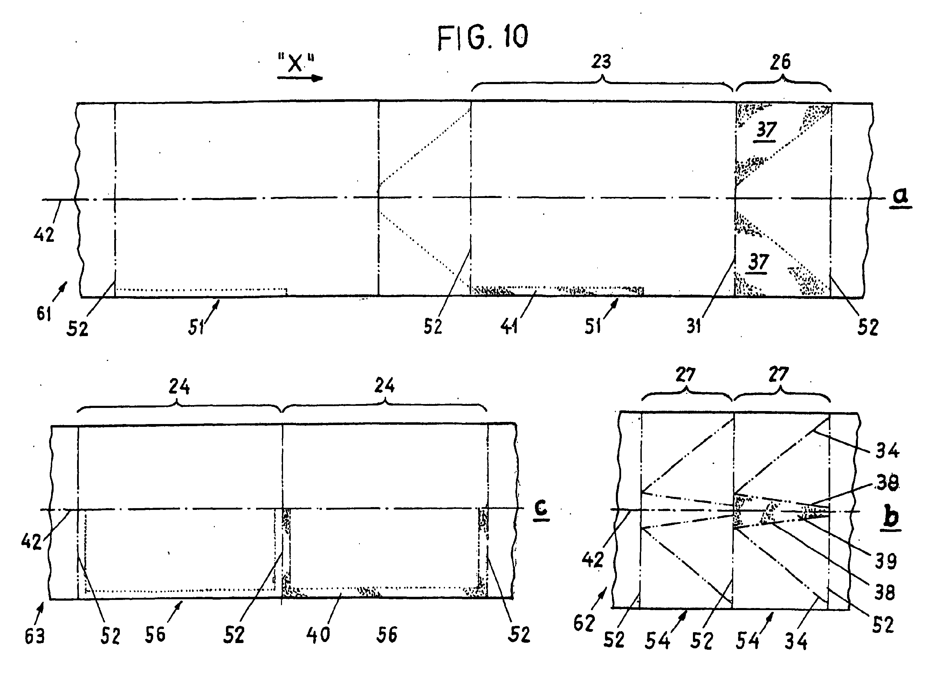

Eine Modifikation der ersten Ausführung des Hilfsmittels wird mit Bezug auf die Figuren 7 bis 13 beschrieben. Das Hilfsmittel besteht aus Hygienepapier und ist als Ableitungsorgan 21 ausgebildet. Das Ableitungsorgan weist die gleichen Merkmale wie die Ausführung nach den Figuren 1 bis 3 auf. Nachfolgend werden lediglich die unterschiedlichen Merkmale beschrieben. Wie die Fig. 7 zeigt,sind eine Mehrzahl von Ableitungsorganen 21 aufeinanderfolgend ausgebildet, wobei der Griffteil 24 des nachfolgenden Ableitungsorgans 21 innerhalb des Ableitungsabschnitts 23 des vorangehenden Ableitungsorgans 21 liegt. Hierzu muss der Griffteil 24 eine geringere Breite aufweisen, wie das aus der Fig.8 ersichtlich ist. Die Ableitungsorgane 21 sind über eine Perforation 52 miteinander so verbunden, dass die Hilfsmittel durch Abreissen vereinzelt werden können. Der Einlassabschnitt 22, der Ableitungsabschnitt 23 und der Griffteil 24 werden aus den in den Figuren 10a bis. 10c dargestellten drei Zuschnitten 51,54,56 gebildet, die jeweils auf einem Streifen aufeinanderfolgend ausgebildet sind.

Wie die Figuren 10a bis 10c zeigen, ist ein erster Zuschnitt 51 durch zwei Perforationen 52 zum Trennen der Ableitungsorgane 21 begrenzt und ist durch eine Rille 31 in den Abschnitt 26 zur Bildung eines Teils des Einlassabschnitts 22 und den Ableitungsabschnitt 23 unterteilt (Fig.10a). Ein zweiter Zuschnitt 54 bildet den Abschnitt 27 zur Bildung des anderen Teils des Einlassabschnitts 22 und ist durch zwei Perforationen 52 begrenzt (Fig.10b). Ein dritter Zuschnitt 56 zur Bildung des Griffteils 24 wird durch die Perforationen 52 begrenzt.

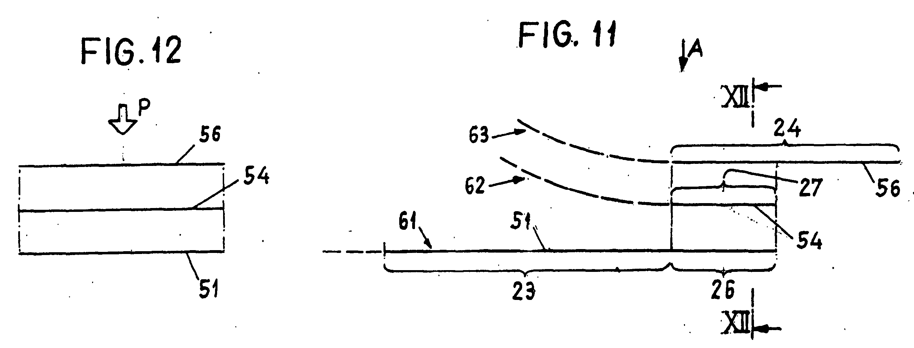

Die Herstellung des vorstehend beschriebenen Ableitungsorgans erfolgt ebenfalls in der Richtung des Pfeiles X mit einem takt-und anschlagfreien Verfahren. Wie die Figuren 11 und 12 zeigen, werden drei Papierstreifen 61, 62 und 63 gestaffelt zugeführt und das Ableitungsorgan 21 mit folgenden Verfahrensschritten hergestellt.

In einem ersten Schritt wird der erste Papierstreifen 61 kontinuierlich zugeführt, entlang der Linie 52 perforiert, mit der Rille 31 versehen und entlang der Linie 52 perforiert. In einem zweiten Schritt wird auf den Abschnitten 37 des Abschnitts 26 ein Klebmittel aufgebracht. Damit ist der erste Zuschnitt 51 vorbereitet (Fig.10a). In einem dritten Schritt wird der zweite Papierstreifen 62 entlang der Linie 52 perforiert, mit den Rillen 34 und 38 versehen und entlang der Linie 52 perforiert. Damit ist der zweite Zuschnitt 54 vorbereitet. In einem vierten Schritt wird der Abschnitt 27 auf den Abschnitt 26 aufgelegt, vom Papierstreifen 62 abgetrennt und die Abschnitte 26, 27 mittels Druck verbunden (Fig.11). In einem fünften Schritt wird ein dritter Papierstreifen 63 mit geringerer Breite entlang den Linien 52 perforiert und im Bereich 40 mit einem Klebmittel versehen. Damit ist der dritte Zuschnitt 56 vorbereitet. In einem sechsten Schritt wird der Abschnitt 24 vom dritten Papierstreifen 63 abgetrennt und auf den Abschnitt 27 so aufgelegt, dass die Trennkante bündig mit der Trennkante des Abschnitts 27 ist und der Griffteil 24 über den zweiten Zuschnitt hinausragt und einen Abschnitt des vorhergehend gebildeten Ableitungsorgans überragt (Fig.11). In einem siebenten Schritt werden der Abschnitt 27 im Abschnitt 39 und der Abschnitt 23 im Bereich 41 mit einem Klebmittel versehen. In einem achten Schritt wird das Ganze entlang der Mittellinie 42 gefaltet, die Abschnitte 23 und 24 auseinander gelegt und miteinander verbunden.

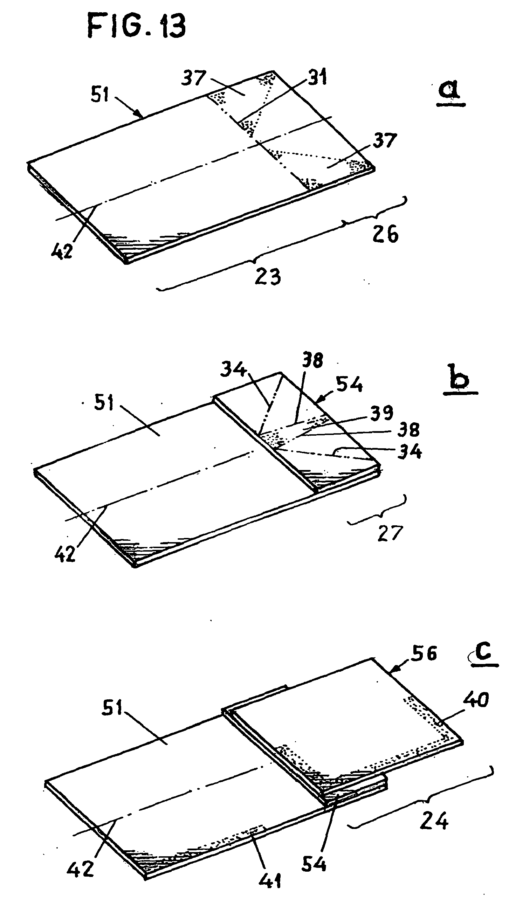

Wie die Fig. 13 zeigt, liegen nach dem Zusammenstellen die mit einem Klebmittel versehenen Bereiche 40 und 41 frei und werden nach dem Falten entlang der Linie 42 aufeinander gelegt und mittels Druck miteinander verbunden.

Die zusammenhängenden Hilfsmittel werden entweder in Rollenform oder im Zickzack laufend gefaltet in einem Spender so angeordnet, dass die Hilfsmittel einzeln entnommen werden können.

Anstelle des Klebmittels 37 können die Bereiche durch eine mechanisch hergestellte Verbindung, z.B. ineinandergreifende Prägungen verbunden sein.

Die Schicht 18 kann durch eine Imprägnierung mit einem umweltfreundlich abbaubaren und bei Berührung mit Wasser rasch auflös- und/oder zersetzbaren Material, z.B. einer Seife, einem Fett oder dgl., gebildet sein. Die Schicht 18 kann ferner ein medizinisches Testmittel enthalten, z.B. einen Indikator, der auf die saure oder basische Beschaffenheit des Urins eines jeweiligen Benutzers reagiert. Derart ausgeführte Ableitungsorgane 21 können daher zugleich als einfache Mittel für entsprechende, z.B. täglich erforderliche medizinische Kontrollfunktionen verwendet werden.

Die Ableitungsorgane 21 dienen zum Erleichtern des Urinierens in einer stehenden Position des jeweiligen Benutzers und zum Ableiten von Urin in eine nicht dargestellte Toilette. Die Hilfsvorrichtung eignet sich insbesondere zur Verwendung in privaten oder anderen Toilettenanlagen, z.B. solchen von Verkehrsmitteln, in denen kein Pissoir vorhanden ist.

Das Einweg- Hilfsmittel ist als gefaltetes Ableitungsorgan 21 mit Wänden aus einem Hygienepapier ausgebildet, welches durch Falten entlang einer Mittellinie gebildet ist. Das Ableitungsorgan 21 enthält einen Einlassabschnitt 22 zur Aufnahme von Urin, einen Ableitungsabschnitt 23 zum Ableiten von Urin in eine Toilette oder dgl. und einen seitlich abstehenden Griffteil 24, der mit dem Einlassabschnitt 22 verbunden ist. Der Einlassabschnitt 22 ist aus zwei wänden mit je zwei gleich ausgebildeten Wandabschnitten 26 bzw. 27 gebildet und kann durch diese zu einem Trichter geformt werden. Eine Mehrzahl von Ableitungsorganen 21 können einzeln oder in Rollenform hergestellt und in einem Spender verpackt werden. Diese Ableitungsorgane können kostengünstig hergestellt werden und ermöglichen eine bequeme, hygienisch vorteilhafte Anwendung sowie eine einfache Entsorgung.

## Patentansprüche

1. Einweg- Hilfsmittel zum Urinieren, mit einem gefalteten Ableitungsorgan (21), das aus flach aneinander liegenden Wänden aus einem in Wasser aufweich- und/oder zersetzbaren Hygienepapier durch Falten entlang einer Mittellinie (42) gebildet ist und das einen mit einer benetzungshemmenden Schicht (18) versehenen Ableitungskanal begrenzt, welches Ableitungsorgan (21) einen zu einem Trichter verformbaren Einlassabschnitt (22) zur Aufnahme von Urin, einen von benetzungshemmenden Mitteln freien Ableitungsabschnitt (23) zum Ableiten von Urin in eine Toilette und einen seitlich abstehenden Griffteil (24) umfasst, **dadurch gekennzeichnet, dass** der Einlassabschnitt (22) aus einer ersten Wand mit zwei Wandabschnitten (26), die einstückig mit zwei Wandabschnitten zur Bildung des Ableitungsabschnitts (23) ausgebildet sind, und aus einer zweiten Wand mit zwei Wandabschnitten (27) gebildet ist, die je mit einem der Wandabschnitte (26) der ersten Wand verbunden sind, dass der Griffteil (24) aus zwei Abschnitten gebildet ist, die je mit einem der Wandabschnitte (27) der zweiten Wand verbunden und durch die Wandabschnitte (26,27) der ersten und der zweiten Wand umschlossen sind und dass der Griffteil (24) über die gefalteten Wandabschnitte (26,27) um einen Abstand hinausragt, der ein automatisches Öffnen des Einlassabschnitts (22) durch Schwenken des Griffteils (24) gewährleistet.

2. Hilfsmittel nach Anspruch 1, **dadurch gekennzeichnet, dass** die Wandabschnitte des Ableitungsabschnitts (23) im Randbereich mindestens teilweise verbunden sind.

3. Hilfsmittel nach Anspruch 1, **dadurch gekennzeichnet, dass** das Ableitungsorgan (21) aus einem einzigen Zuschnitt (30) gebildet ist.

4. Hilfsmittel nach Anspruch 1, **dadurch gekennzeichnet, dass** das Ableitungsorgan (21) aus einem ersten, einem zweiten und einem dritten Zuschnitt (51, 54, 56) gebildet ist.

## Claims

1. A disposable urinary aid comprising a folded discharge unit (21), which is formed by means of folding along a center line (42) from walls lying adjacent to one another in a flat manner and being made of a hygienic paper, which is capable of being moistened and/or decomposed in water and which defines a discharge channel, which is provided with a wettability-limiting layer (18), said discharge unit (21) comprising an inlet section (22), which can be deformed into a funnel, for receiving urine, a discharge section (23) for discharging urine into a toilet, which is free from wettability-limiting means and a laterally protruding grip part (24), **characterized in that** the inlet section (22) is formed from a first wall comprising two wall sections (26), which are embodied in one piece comprising two wall sections for forming the discharge section (23) and of a second wall comprising two wall sections (27), each of which are connected to one of the wall sections (26) of the first wall, **in that** the grip part (24) is formed from two sections, each of which are connected to one of the wall sections (27) of the second wall and are enclosed by means of the wall sections (26, 27) of the first and the second wall and that the grip part (24) protrudes beyond the folded wall sections (26, 27) by a distance, which ensures an automatic opening of the inlet section (22) by pivoting the grip part (24).

2. The aid according to claim 1, **characterized in that** the wall sections of the discharge section (23) are at least partly connected in the edge region.

3. The aid according to claim 1, **characterized in that** the discharge unit (21) is formed from a single pre-cut part (30).

4. The aid according to claim 1, **characterized in that** the discharge unit (21) is formed from a first, a second and a third pre-cut part (51, 54, 56).

## Revendications

1. Dispositif auxiliaire jetable pour uriner, avec un organe d'évacuation plissé (21), qui est formé de parois adjacentes plates en un papier hygiénique ramollissable et/ou destructible dans l'eau, par des plis le long d'un ligne médiane(42) et qui délimite un canal d'évacuation muni d'une couche (18) inhibant le mouillage, lequel organe d'évacuation (21) comprend une section entrée (22) déformable en direction d'un entonnoir, pour recevoir de l'urine, une section d'évacuation (23) exempte de produits inhibant le mouillage, pour évacuer de l'urine dans des toilettes et un élément formant poignée (24) débordant latéralement, **caractérisé en ce que** la section entrée (22) est réalisée en une première paroi avec deux sections de paroi, qui sont réalisées en monobloc avec deux sections de paroi pour créer la section d'évacuation (23) et en une deuxième paroi avec deux sections de paroi (27) dont chacune est reliée avec l'une des sections de paroi (26) de la première paroi, **en ce que** l'élément formant poignée (24) est réalisé en deux sections, dont chacune est reliée avec l'une des sections de paroi (27) de la deuxième paroi et qui sont entourées par les sections de paroi (26, 27) de la première et de la deuxième paroi et **en ce que** l'élément formant poignée (24) saillit par-dessus les sections de paroi plissées (26, 27) d'une distance qui assure une ouverture automatique de la section d'entrée (22) par pivotement de l'élément formant poignée (24).

2. Dispositif auxiliaire selon la revendication 1,
**caractérisé en ce que**,
les sections de paroi de la section d'évacuation (23) sont au moins partiellement reliées dans la zone de bordure.

3. Dispositif auxiliaire selon la revendication 1, **caractérisé en ce que** l'organe d'évacuation (21) est formé à partir d'un seul coupon (30).

4. Dispositif auxiliaire selon la revendication 1, **caractérisé en ce que** l'organe d'évacuation (21) est formé à partir d'un premier, d'un deuxième et d'un troisième coupons (51, 54, 56).
